Europäisches Patentamt

European Patent Office

Office européen des brevets

(11) Publication number: **0 216 195**
**B1**

(12) **EUROPEAN PATENT SPECIFICATION**

(45) Date of publication of patent specification: **13.06.90**

(21) Application number: **86112005.3**

(22) Date of filing: **29.08.86**

(51) Int. Cl.⁵: **C 12 N 15/00,** C 12 P 21/00, A 61 K 39/245 // (C12N15/00, C12R1:19)

(54) Recombinant plasmid inserted with herpes simplex virus gene.

(30) Priority: **30.08.85 JP 192916/85**

(43) Date of publication of application: **01.04.87 Bulletin 87/14**

(45) Publication of the grant of the patent: **13.06.90 Bulletin 90/24**

(84) Designated Contracting States: **AT BE CH DE FR GB IT LI NL SE**

(56) References cited:
EP-A-0 100 521
EP-A-0 101 655
EP-A-0 168 662
EP-A-0 170 169

Virology, vol. 133, no. 2, March 1984, New York; D.J. Bzik et al.:"Nucleotide Sequence Specifying the Glycoprotein Gene, gB, of Herpes Simplex Virus Type 1",pages 301-314

(73) Proprietor: **Juridical Foundation The Chemo-Sero-Therapeutic Research Institute 668, Okubo Shimizu-machi Kumamoto-shi Kumamoto-ken (JP)**

(72) Inventor: **Nozaki, Chikateru No. 622-8, Shinchi Shimizu-machi Kumamoto-shi Kumamoto-ken (JP)**
Inventor: **Nakatake, Hiroshi No. 402-1, Takahira Shimizu-machi Kumamoto-shi Kumamoto-ken (JP)**
Inventor: **Kino, Yoichiro No. 2-142, Musashigaoka Kumamoto-shi Kumamoto-ken (JP)**
Inventor: **Eto, Tatsuo No. 29-27, Tsunoura-machi Kumamoto-shi Kumamoto-ken (JP)**
Inventor: **Makizumi, Keiichi No. 137, Yamamuro Shimizu-machi Kumamoto-shi Kumamoto-ken (JP)**
Inventor: **Ohtomo, Nobuya No.6-22-29, Shimasaki Kumamoto-shi Kumamoto-ken (JP)**

(74) Representative: **Vossius & Partner Siebertstrasse 4 P.O. Box 86 07 67 D-8000 München 86 (DE)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

## Description

The present invention relates to a novel recombinant plasmid inserted with herpes simplex virus gene, i.e. a recombinant plasmid which is useful for the production of a herpes simplex virus protein which is in turn useful for the production of a herpes simplex vaccine for the effective prophylaxis of herpes simplex virus infections.

More particulary, the present invention relates to a recombinant plasmid which is obtained by inserting a specific gene (gB gene) of herpes simplex virus (hereinafter, referred to as "HSV"), i.e. the HSVgB gene, into a shuttle vector which can replicate in both *Escherichia coli* and yeast downstream of the appropriate promoter region carried on the vector. The invention further relates to a novel transformed yeast which is produced by transforming a yeast with said recombinant plasmid. Additionally, the invention relates to a method for producing HSV proteins, particularly HSV membrane proteins, in high yield and high purity by cultivating the transformed yeast.

In developed countries, the population having immunity against HSV has recently decreased, and hence, serious problems arise in HSV infections such as herpes genitalis, neonatal herpes infection and herpes encephalitis in these countries. In order to prevent such HSV infections, a vaccine is useful. There have already been proposed some vaccines such as attenuated vaccines comprising attenuated HSV and inactivated vaccines containing HSV DNA. It is known, however, that HSV includes some problems such as latent infection and carcinogenicity. The conventional attenuated vaccines and inactivated vaccines include such side effects and hence are not preferable from a practical viewpoint.

Cells infected by HSV produce several glycoproteins [e.g. gB, gC, gD, gE, etc., the nomenclature of gA and gB has been standardized as "gB" at the International Herpes Virus Workshop (Oxford, England) in 1983]. Since it has been found that these glycoproteins have an important function as an antigen for inhibiting the HSV infection, various studies have been done on component vaccines comprising these glycoproteins. For instance, Cappel et al. have reported that glycoproteins extracted from HSV-infected cells or virus particles are effective as an antigen for preventing HSV infections [of. Cappel et al., Arch Virol., *73*, 61, (1982)]. However, the component vaccine comprising such glycoproteins extracted from HSV-infected cells or virus particles contains a variety of proteins origined from the host cells and hence causes side effects due to the extra proteins. In order to obtain a suitable component vaccine having no side effects, it is necessary to obtain highly purified glycoproteins. The present inventors aimed at gB which is one of the glycoproteins and have experimentally confirmed that the highly purified gB is very effective in mice [cf. Kino, Cellular Technology, *3*, 120 (1984)].

The glycoprotein gB is usually produced by inocculating a virus into culture cells and then culturing the cells. However, this method is troublesome because of handling infectious material and performing complicated experimental steps. Furthermore, it is impossible to assure the complete removal of viral DNA carrying carcinogenic DNA sequences. Thus, it is very difficult to produce a practically safe component vaccine using the natural glycoprotein gB.

The present inventors had intensively studied on a safe vaccine effective for herpes simplex virus infections. They had found that the desired HSV proteins (i.e. HSVgB) could be produced by a genetic engineering technique. This technique comprises isolating the HSVgB gene and inserting the isolated HSVgB gene into a specific plasmid vector containing yeast DNA sequences, *E. coli* DNA sequences and the expression control region of the repressible acid phosphatase gene of yeast. The inserted HSVgB gene is under the control of said phosphatase promotor. Yeast cells are then transformed with the new recombinant DNA molecule and cultured (cf, Japanese Patent Application Nos. 151766/1984 and 262465/1984, U.S. Serial No. 755,776, European Patent Application No. 85109042.3, and Canadian Patent Application No. 487034).

According to the process disclosed in the above applications highly pure HSVgB can be produced. This is suitable for the preparation of a vaccine by a genetic engineering technique, but it is still desired to develop an improved process suitable for the production of the desired HSVgB in higher yield.

The present inventors have further developed a process for the production of the desired HSVgB in higher yields. According to this process a recombinant plasmid is constructed by inserting a specific HSVgB gene, i.e. a herpes simplex virus gene where an N-terminal part of the gene is removed which includes a sequence encoding the so-called signal peptide into the vector as mentioned hereinbefore. Then a yeast is transformed with the recombinant plasmid and the transformant is cultured.

An object of the present invention is to provide an improved recombinant plasmid which comprises inserting a herpes simplex virus gB gene having lost a part of the N-terminus including a sequence encoding the signal peptide into a plasmid vector containing yeast DNA sequences, *E. coli* DNA sequences and a promoter region downstream and under the control of said promoter. Another object of the invention is to provide a transformed yeast obtained by transforming yeast with the recombinant plasmid as set forth above. A further object of the invention is to provide a process for the production of the desired HSVgB by culturing the transformed yeast. These and other objects and advantages of the invention will be apparent to skilled persons from the following description.

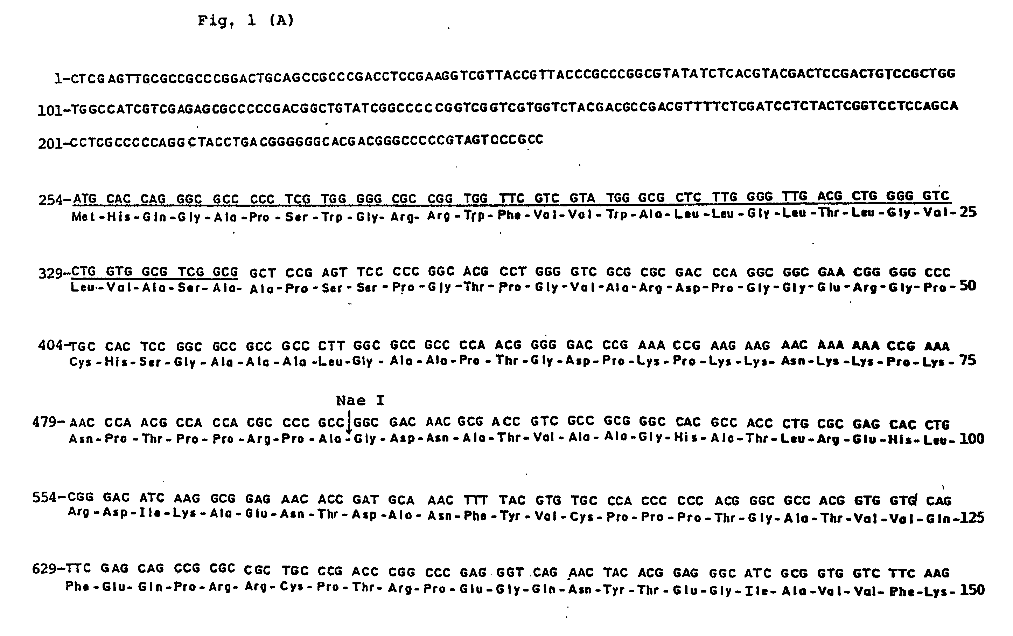

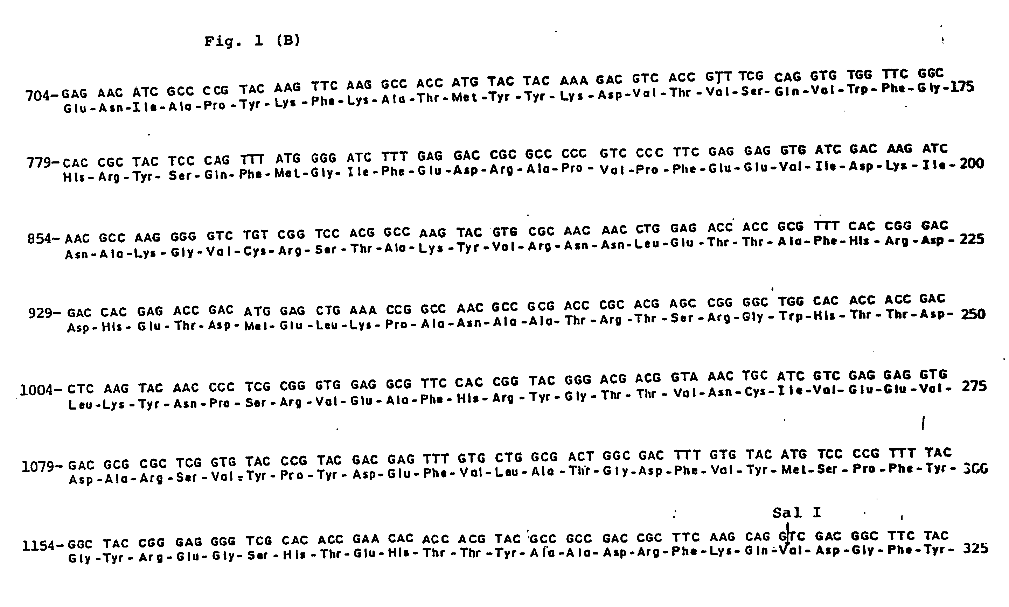

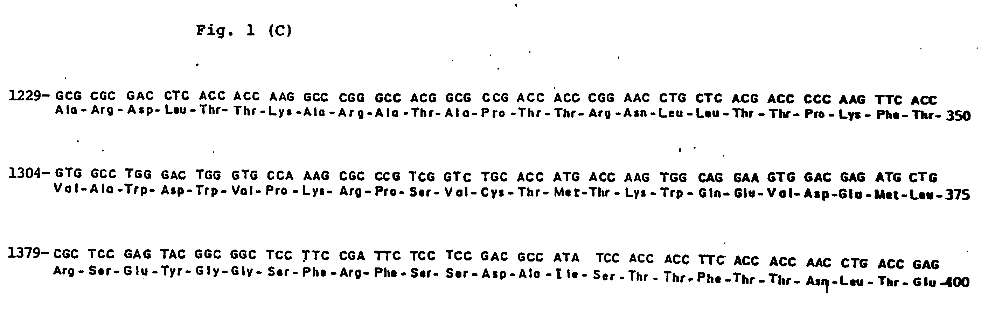

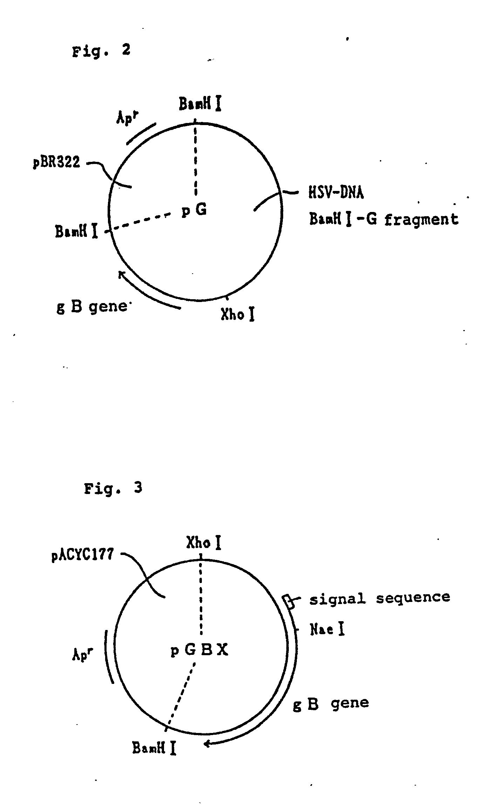

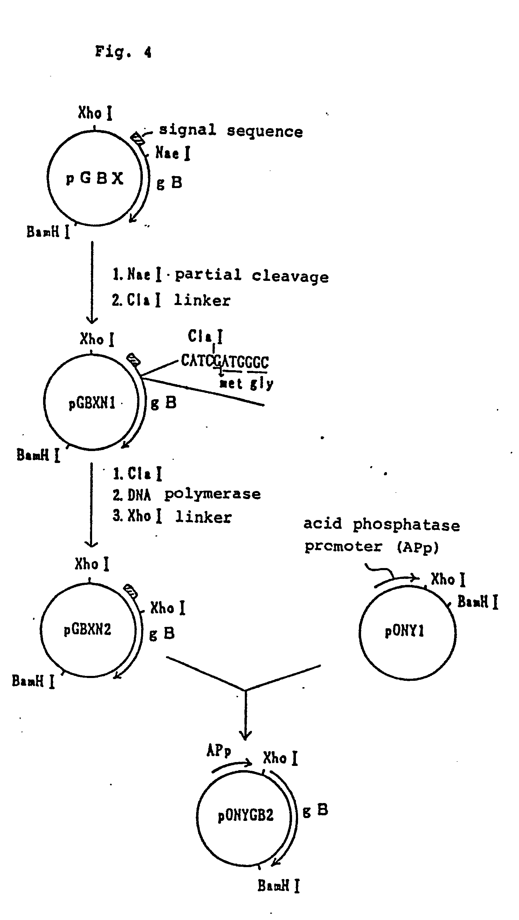

Fig. 1 shows the base sequence of HSVgB gene used in the present invention. Fig. 2 shows a structure of plasmid pG containing the BamHI-G fragment of HSV DNA used in the present invention. Fig, 3 shows the structure of plasmid pGBX used in the present invention containing a HSVgB gene having a signal peptide-encoding region. Fig. 4, Fig. 5 and Fig. 6 shows steps for the construction of various recombinant plasmids from plasmid pGBX of the present invention.

The HSVgB gene has a base sequence as shown in Fig. 1 which is also disclosed in the above-mentioned patent application. The N-terminus of the base sequence, encodes the so-called signal peptide which consists of 30 amino acids (in the figure, the underlined region). According to the present invention an HSVgB gene fragment where the sequence encoding the N-terminal 30 amino acids is removed, or an HSVgB gene fragment where the above-mentioned region and further downstream sequences therefrom are removed (for instance, an HSVgB gene fragment having lost a sequence encoding the N-terminal 83 amino acids which can be cleaved by Nae I, or an HSVgB gene fragment having lost a sequence encoding the N-terminal 320 amino acids which can be cleaved by Sal I), are used for the construction of a plasmid. Said HSVgB gene fragments are inserted into a shuttle vector under the control of a yeast promoter, and a yeast is transformed with the recombinant plasmid obtained. Then the transformed yeast is cultured to produce in a high yield the desired HSVgB proteins having antigenic and imunogenic biological activities.

The plasmid vector suitable for the recombination of the herpes simplex virus gene without the DNA sequence encoding the signal peptide is, for example, a vector containing a yeast DNA sequence, an E. coli DNA sequence and the expression control region of the repressible acid phosphatase gene of yeast. Other plasmid vectors carrying a different promoter having a similar function can also be used.

The recombinant plasmid of this invention, the transformation of a yeast with said plasmid, and the production of HSVgB therewith are explained in more detail below.

(1) Shuttle vector

In the present invention, the herpes simplex virus gB gene without the sequence encoding the signal peptide is inserted into a shuttle vector being replicable in both yeast and E. coli and carrying a promotor which can control the expression of HSVgB. The HSVgB gene is inserted downstream of the promotor region as mentioned hereinbefore.

A suitable shuttle vector used in the present invention is a plasmid vecor which contains both a yeast gene, i.e. important yeast DNA sequences, and an E. coli gene, i.e. important E, coli DNA sequences, and which carries the repressible acid phosphatase gene of yeast, e.g. of Saccharomyces cerevisiae. The shuttle vector is constructed as disclosed below.

The yeast DNA sequence contains a DNA sequence which is necessary for replication of a plasmid in yeast independently from the chromosomes, for instance, a DNA sequence necessary for replication of the yeast (ars 1) and a DNA sequence necessary for the replication of 2 μm DNA (2 μ ori).

Optionally it contains a gene useful as a selective marker of the transformed yeast, for example, a leucine-producing gene, a histidine-producing gene, a trypthophane-producing gene, an uracil-producing gene, an adenine-producing gene, or the like. These genes may be used alone or in combination of two or more.

The E. coli DNA sequence contains a DNA sequence necessary for the replication of the plasmid within cells of E. coli, for example, a DNA sequence of a replication initiating region of plasmid of Col EI, and preferably it contains a gene useful as a selective marker of the transformed E. coli. The selective marker includes, for example, an ampicillin-resistance gene, a kanamycin-resistance gene, a tetracycline-resistance gene, a chloramphenicol-resistance gene, or the like. These genes may be used alone or in combination of two or more. Conventionally used E. coli. DNA is pBR322 which contains an ampicillin resistance gene and a tetracycline-resistance gene.

The shuttle vector used in the present invention is characteristic in that it carries the repressible acid phosphatase promoter of the yeast. The acid phosphatase promoter is usually a promoter of a polypeptide of 60,000 dalton (P60) which constitutes the phosphatase.

Suitable examples of the shuttle vector are produced from shuttle vector pAT 77 whereon a yeast DNA sequence containing ars 1, 2 μori and the leucine-producing gene (Leu 2) as the yeast gene is combined with E. coli. plasmid pBR322, i.e. by treating the shuttle vector pAT 77 with an exonuclease BAL 31 to delete a part or whole of the structural gene of acid phosphatase and further optionally various regions upstream therefrom up to −100 bp, preferably from +1 to −50 bp. A representative example is shuttle vector pAM 82 wherein the upstream region is deleted up to −33 bp. The method for the production of these shuttle vectors is disclosed in Japanese Patent First Publication No. 31799/1984. The shuttle vectors pAT 77 and pAM 82 carried by Saccharomyces cerevisiae (i.e. Saccharomyces cerevisiae AH 22/pAT 77 and Saccharomyces cerevisiae AH 22/pAM 82, respectively) have been deposited at Fermentation Research Institute, Agency of Industrial Science and Technology, Japan under Budapest Treaty as "FERM BP—324" and "FERM BP—313", respectively.

The shuttle vector pAM 82 has a Xho I site downstream of the acid phosphatase promoter and also a Pvu II site further downstream thereof. In order to insert the HSVgB gene of this invention into the vector, pAM 82 is cleaved with the restriction enzyme Pvu II, producing flush ends, and a BamHI linker is bound to said ends. The combined fragment is recircularized and plasmid pONY1 is obtained. This plasmid pONY1 can express an exogenous DNA sequence in pure form under the control of the acid phosphatase promotor. Plasmid pONY1 can be readily cleaved with the restriction enzymes BamHI and Xho I, and hence, it is suitable for inserting desired genes, i.e. exogenous DNA sequences.

(2) Production of HSVgB gene-containing fragments. As to the gB gene of HSV (KOS strain: P.S.E.B.M., 1964, Vol. 115), the position on the virus DNA (0.348—0.366 map units) and the base sequence have been determined by Bjik et al. [cf. David J. Bjik et al., Virology. *133*, 301—314 (1984)].

The HSVgB gene to be inserted into a shuttle vector of the present invention is contained in a fragment of about 8 kb (0.345—0.399 map units). It is obtained by treating HSV DNA with the restriction enzyme BamHI. Said fragment is hereinafter referred to as "BamHI-G fragment".

The HSV-gB-containing fragment can be prepared by cleaving HSV DNA with BamHI. The obtained BamHI-G fragment can be cloned as follows.

Virus DNA is isolated from HSV which have been grown on Vero cells. The virus DNA is cleaved with the restriction enzyme BamHI. Then the resulting BamHI-G fragment is isolated and extracted by subjecting it to agarose gel electrophoresis. The BamHI-G fragment is ligated in the presence of $T_4$ ligase with the *E. coli* plasmid pBR322 which was previously treated with BamHI. *E. coli* X1776 is transformed with the above reaction mixture. Among the transformants thus obtained, a strain having ampicillin resistance (Ap$^r$) and tetracycline sensitivity (Tc$^s$) is selected and amplified. It contains the plasmid pG which carrying the BamHI-G fragment. This plasmid pG has the structure shown in the accompanying Fig. 2.

The plasmid pG thus obtained is cleaved with both BamHI and Xho I to give a fragment (3.5 kb) containing the HSVgB gene. The fragment thus obtained is isolated by extraction. Then it is ligated in the presence of $T_4$ ligase with a fragment (2.6 kb) of *E. coli* pACYC177 which was previously treated with BamHI and Xho I. *E. coli* is transformed with the above reaction mixture, and the resulting transformant having ampicillin resistance is propagated.

Plasmid pGBX containing the HSVgB gene is obtained from the propagated cells. This plasmid pGBX has the structure shown in the accompanying Fig. 3.

(3) Construction of HSVgB gene-expression plasmid

As is mentioned hereinbefore, the present invention provides a recombinant plasmid obtained by recombining an HSVgB gene fragment without the sequence encoding the signal peptide of the HSVgB gene or an HSVgB gene fragment without the sequence encoding said signal peptide and a downstream region of it into a shuttle vector. Using the recombinant plasmid, there can be produced a herpes simplex virus protein suitable for the production of a vaccine effective for the prophylaxis of herpes simplex virus infections. The HSVgB gene-expression plasmid can be constructed from the above plasmid pGBX by removing various regions containing the sequence encoding the signal peptide which vary depending on the cleaving sites for restriction enzymes of the HSVgB gene as follows.

(a) Removing a-sequence encoding the N-terminal 83 amino acids (cf. Fig, 4):

Firstly, the plasmid pGBX is partially cleaved with the restriction enzyme I, a Cla I linker (CATCGATG) is ligated thereto with $T_4$ ligase and then it is recircularized. Among the produced plasmids, there is selected a plasmid wherein only the Nae I site corresponding to the 83rd amino acid from the N-terminal methionine is converted into a Cla I site (this plasmid is hereinafter referred to as "plasmid pGBXN1"). By this procedure, the methionine-coding ATG is added at one position upstream of the 84th amino acid from the N-terminal methionine of gB.

The plasmid pGBXN1 obtained above is cleaved with the restriction enzyme Cla I. The linearized molecule is treated with DNA polymerase to create flush ends. An Xho I linker is bound thereto with $T_4$ ligase and the molecule is recircularized. The thus obtained plasmid (referred to as "pGBXN2") has a structure in which the Cla I site of the plasmid pGBXN1 is exchanged with a Xho I site.

The plasmid pGBXN2 is cleaved with Xho I and BamHI, and from the resulting fragments, a fragment (2.9 kb) containing no gB signal sequence-coding region is selected. It is inserted into the shuttle vector pONY1 which was previously treated with Xho I and BamHI using $T_4$ ligase.

The desired plasmid containing an HSVgB gene fragment without the sequence encoding the signal peptide downstream of the acid promoter is obtained (the plasmid is hereinafter referred to as "pONYGB2").

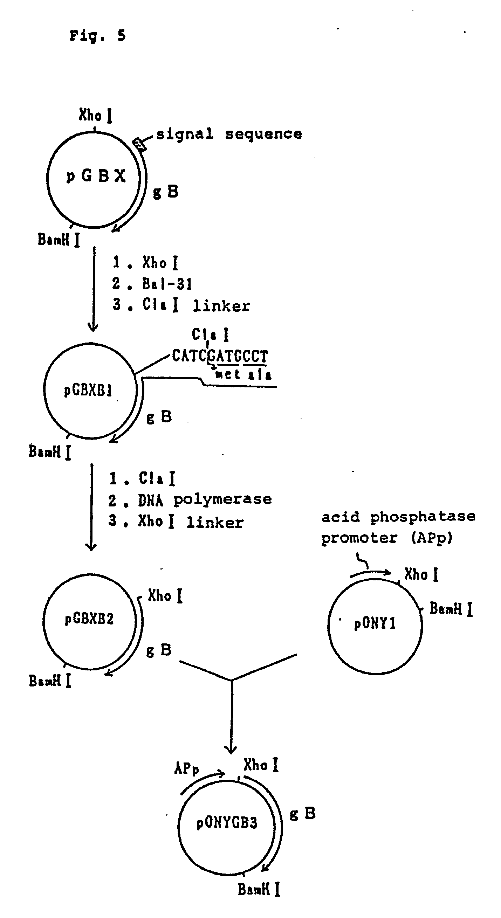

(b) Removing a sequence encoding the N-terminal 30 amino acids (signal peptide-encoding sequence (cf. Fig. 5):

The plasmid pGBX is cleaved with the restriction enzyme Xho I, and in order to remove the signal sequence of gB gene, it is treated with exonuclease Bal 31. The DNA treated with Bal 31 is bound to a Cla linker (CATCGATG) using $T_4$ ligase and then recircularized. Among the plasmids, there is selected a plasmid (hereinafter referred to as "pGBXB1") wherein the sequence encoding the signal peptide of the gB gene is removed and further the ATG of the inserted Cla I linker is in the same frame as the codons encoding the amino acids of the gB gene downstream thereof.

The plasmid pGBXB1 obtained above is cleaved with the restriction enzyme Cla I. The linearized molecule is treated with DNA polymerase to create flush ends. An Xho I linker is bound thereto (CCTCGAGG) with $T_4$ ligase and the molecule is recircularized. The thus obtained plasmid (referred to as 'pGBXB2") is cleaved with Xho I and BamHI. A 3 Kb DNA fragment containing the gB gene is isolated and then inserted into the shuttle vector pONY1 which was previously treated with Xho I and BamHI using $T_4$ ligase. The desired plasmid containing the gB gene downstream of the acid phosphatase promoter is obtained (the plasmid is hereinafter referred to as "pONYGB3").

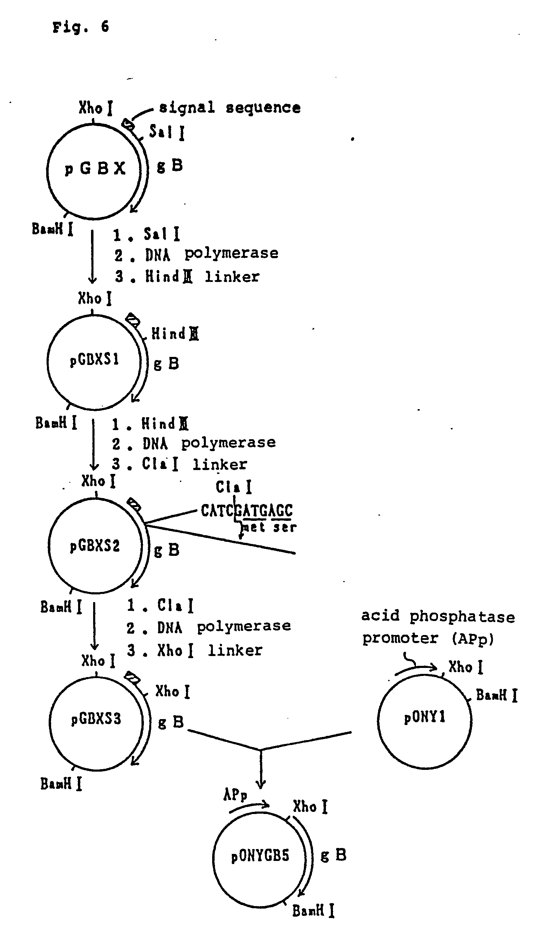

(c) Removing a sequence encoding the N-terminal 320 amino acids (cf. Fig. 6):

The plasmid pGBX is cleaved with the restriction enzyme Sal I. The linearized molecule is treated with DNA polymerase to create flush ends. A Hind III linker is bound thereto (ACAAGCTTGT) using $T_4$ ligase and

4

the molecule is recircularized. The thus prepared plasmid (referred to as 'pGBXS1) is cleaved with Hind III. Its ends are converted into flush ends with DNA polymerase. The linear molecule is bound to a Cla I linker (CATCGATG) using $T_4$ ligase and then recircularized. In the thus prepared plasmid (referred to as "pGBXS2"), the ATG of the inserted Cla I linker is in the same frame as the codons encoding the amino acids of the gB gene downstream thereof.

The plasmid pGBXS2 obtained above is cleaved with the restriction enzyme Cla I and its ends are converted into flush ends with DNA polymerase. The linear molecule is bound to an Xho I linker (CCTCGAGG) with $T_4$ ligase and then recircularized. The thus obtained plasmid (referred to as "pGBXS3") is cleaved with Xho I and BamHI. A 2.2 Kb DNA fragment containing the gB gene is isolated and then inserted into the shuttle vector pONY1 which was previously treated with Xho I and BamHI using $T_4$ ligase. The desired plasmid containing the gB gene downstream of the acid phosphatase promoter is obtained (the plasmid is hereinafter referred to as "pONYGB5").

(4) Transformation of yeast

The yeast to be transformed includes a mutant strain of yeast which is complemental with the selective marker gene of the transformed yeast carried out on the plasmid, for example, a leucine-requiring mutant, *Saccharomyces cerevisiae* AH 22 [a, leu 2, his 4, Can 1 (Cir⁺)] (cf. Hinnen, A. et al., Proc. Natl. Acad. Sci., U.S.A., *75*, 2157—2161, 1978) or *Saccharomyces cerevisiae* AH 22 pho 80 [a, leu 2, his 4, Can 1 (Cir⁺)] (cf. Thoe, A. et al., J. Bacteriol., *145*, 221—232, 1981). After amplification in *E. coli*, the isolated recombinant plasmid is transformed into the mutant strain of yeast in a usual manner, for example, by mixing the plasmid DNA with calcium treated spheroblasts of the yeast cells.

The desired transformed yeast is selected and isolated from the yeast culture based on the expression of a gene complemental with the mutation of the host yeast carried on the vector, for example, expression of a leucine-producing gene.

In addition to the above-mentioned leucine-requiring yeast strain, various mutant strains such as a histidine-requiring strain, a tryptophane-requiring strain, an uracil-requiring strain, an adenine-requiring strain, or the like can be used.

(5) Culture of transformed yeast and production of HSVgB

The transformed yeast obtained above is cultured in a usual manner in a medium containing phosphoric acid.

The culture cells in logarithmic growth phase are transferred to an inorganic phosphate free medium. Then they are cultured under a condition that the acid phosphatase promoter is not repressed. After the culture, the produced cells are collected and lysed in a usual manner to give a lysed cell solution containing a large amount of the desired HSVgB.

Depending on the kind of yeast, for instance, when pho 80 mutant strain is used, the culture is not necessarily required to be carried out under the condition that the acid phosphatase promoter is not repressed, but may be done under a usual condition to give directly the desired HSVgB in a large amount.

The HSVgB can be purified by conventional purification methods. For instance, the protein-containing solution is passed through a column packed with a gel carrying an anti-gB antibody, and then the absorbed gB is eluted with 3 M KSCN.

The gB thus obtained has the same immunological properties as the natural gB obtained from HSV-infected cells and can be used for the preparation of HSV vaccines and diagnostic reagents.

The recombinant DNA, transformed yeast, and the production of HSVgB of the present invention are illustrated by the following example, but should not be construed to be limited thereto.

Example

(1) Preparation of DNA containing HSVgB gene:

(i) Preparation of HSV—1 DNA:

Vero cells (about $5 \times 10^8$ cells) are infected with 0.5—1 PFU/cell of HSV type 1 (HSV—1) KOS strain and the cells are cultured at 37°C for 20—24 hours. When the cells are sufficiently modified, the culture mixture is centrifuged at 90 000 x g for 1 hour to separate the infected cells and the supernatant, and thereby pellets of infected cells (2—3 ml) are isolated. The pellets are suspended in a phosphate buffered saline solution (hereinafter, referred to as "PBS") (pH 7.2, 6 ml). The suspension is subjected to ultrasonic treatment (9 KHz, 200 W, for 5 minutes) or to freezing — thawing [repeating three times freezing at −50°C (with acetone-dry ice) and thawing at 37°C] to fracture the cells, and the cell residue is removed by centrifugation at low speed (1000 x g, 20 minutes). The solution thus obtained is layered on glycerol cushion (5%, 40%) and then centrifuged at 144000 x g for one hour. The pellets (0.5—1 ml) thus obtained are suspended in PBS (1—2 ml) and are treated with DNase (10 μg/ml) and RNase (0.3 mg/ml) at 37°C for one hour, and to the reaction mixture is added 1/5 volume of 5 x STEP [a mixture of 0.5% SDS (sodium dodecylsulfate), 50 mM Tris-HCl (pH 7.5), 0.4 M EDTA, and 0.1 % proteinase K], and the mixture is reacted at 50°C for 30 minutes. The resulting solution is extracted with an equivolume of phenol, phenol-chloroform (1:1) and chloroform in this order to give an aqueous layer containing DNA.

The aqueous layer is dialyzed against TE buffer (20 mM Tris-HCl, 1 mM EDTA, pH 7.5), and thereto is added cold ethanol to precipitate the DNA. The DNA is separated by filtration, dried in vacuum, and then dissolved in an aqueous cesium chloride (Rf 1.3885, incorporated with ethidium bromide (0.04%) and lauroyl sarcosinate (0.4%), 5 ml]. The mixture is centrifuged at 200000 x g for 72 hours to form a band of HSV—1 DNA. The band is recovered and is washed with isopropyl alcohol to remove ethidium bromide, and then dialyzed against TE buffer. Cold ethanol is added thereto to precipitate HSV—1 DNA.

(ii) Cloning of BamHI-cleaved G fragment of HSV—1 DNA:

The HSV—1 DNA obtained above (about 100 µg) is treated with a restriction enzyme BamHI in a mixture (0.75 ml) off 73 mM Tris-HCl (pH 8.0), 7 mM $MgCl_2$, 100 mM NaCl and 2 mM 2-mercaptoethanol at 37°C for 6 hours, and each fragment is separated by 0.7% agarose electrophoresis to cut out a gel corresponding to G fragment (0.345—0.399 map units), from which the G fragemnt is electrophoretically recovered.

E. coli pBR322 plasmid 100 ng which is obtained by cleaving with a restriction enzyme BamHI in the same manner as described above is reacted with the above G fragment (about 2 µg) in a mixture of 50 mM Tris-HCl (pH 7.9), 10 mM $MgCl_2$, 20 mM dithiothreitol and 1 mM ATP by using $T_4$ DNA ligase at 16°C for about 16 hours.

The above reaction mixture is added to a liquid of E. coli (0.1 ml) which is prepared by treating a culture broth of E. coli x 1776 (cf. Curtiss, R III, "Molecular Cloning of Recombinant DNA" Ed, Scott, W. A. and Werner, R., page 99, Academic Press, 1977) by the procedure as described in Norgard, M. V., Gene, 3, 279 (1978), and the mixture is mixed well and allowed to stand at 0°C for 45 minutes. The mixture is applied onto an agar plate containing ampicillin (100 µ/ml) and then incubated at 37°C overnight. The resulting colonies are applied onto both an agar plate containing ampicillin (100 µg/ml), and an agar plate containing tetracycline (100 µg/ml), and are incubated likewise. The colonies which grow only on the agar plate containing ampicillin are selected. pBR322 has an ampicillin-resistance gene and a tetracycline-resistance gene, but when it is inserted with HSV—1 DNA fragment at the BamHI site of the tetracycline-resistance gene, it loses the tetracycline-resistance. Accordingly, the selected colonies contain a recombinant DNA of BamHI-G fragment of pBR322-HSV DNA.

From the colonies this selected, a plasmid is prepared by the procedure as described by K. Matsubara (J. Virol., 16, 479, 1975). The plasmid thus prepared is subjected to restriction map analysis by treating with various restriction enzymes (e.g. BamHI, Bst EII, Kpn I, Sal I, Sst I, Xho 1) to obtain a recombinant DNA of pBR322-BamHI-G fragment wherein BamHI-G fragment of HSV—1 DNA is inserted into pBR322 (hereinafter, referred to as "plasmid pG").

(iii) Preparation of plasmid pGBX:

The plasmid pG (10 µl) obtained in the above (ii) is added to a mixture (100 µl) of 6 mM Tris—HCl (pH 7.5), 6 mM $MgCl_2$, 6 mM 2-mercaptoethanol and 150 mM NaCl, and thereto are added the restriction enzymes BamHI (10 units) and Xho I (10 units), and the mixture is reacted at 37°C for 2 hours. A 3.5 kb fragment DNA is isolated from the reaction mixture by 1% agarose gel electrophoresis in accordance with the method disclosed by Yasusuke Takagi, "Manual for Procedure of Genetic Engineering", pages 33—34, and the 3.5 kb fragment DNA (100 ng) is reacted with E. coli plasmid pACYC 177 (which is cleaved with BamHI and Xho I) (10 ng) in a mixture (10 µl) of $T_4$ ligase (0.1 unit), 6.6 mM $MgCl_2$ and 10 mM dithiothreitol at 16°C for 8 hours. By using the above reaction mixture, E. coli x1776 is transformed in the same manner as described hereinbefore, and ampicillin resistant cells are selected from the resulting transformants. From the cells thus selected, a plasmid pGBX containing gB gene is prepared by the procedure as described by K. Matsubura (J. Virol., 16, 479, 1975).

(iv) Preparation of plasmid pGBXN2:

The above plasmid pGBX is treated in the following manner, whereby only the Nae I site at about 250 bp downstream from the gB gene translation initiation codon ATG is converted into a Cla I site.

The plasmid pGBX (2µg) is treated with Nae I (0.1 unit) in a mixture of 6 mM Tris-HCl (pH 7.5), 50 mM NaCl, 6 mM $MgCl_2$ and 6 mM 2-mercaptoethanol and the mixture is subjected to precipitation with ethanol. The DNA thus obtained is added to a mixture (50 µl) of $T_4$ DNA polymerase (0.1 unit), 200 µM dATP, dCTP, dGTP, dTTP, 67 mM Tris-HCl (pH 8.6), 6.7 mM $MgCl_2$, 10 mM 2-mercaptoethanol, and 16.7 mM $(NH_4)_2SO_4$, and the mixture is reacted at 37°C for 30-minutes and then subjected to treatment with phenol and precipitation with ethanol. The DNA thus obtained (1 pmole) is added to a mixture (10 µl) of $T_4$ ligase (0.1 unit), 66 mM Tris-HCl (pH 7.6), 6.6 mM $MgCl_2$, 10 mM dithiothreitol, 66 µM ATP and 10 pmole Cla I linker (CATCGATG), and the mixture is reacted at 16°C for 8 hours.

E. coli x1776 is transformed with the reaction mixture obtained above in the same manner as described hereinbefore. A large number of clones which grow on an amplicillin-containing plate are selected, and the plasmids are prepared from the selected cells in the same manner as described above. The plasmids are each treated with the restriction enzyme Cla I and BamHI, and thereby there is selected the desired plasmid pGBXN1 by the patterns in electrophoresis. In the plasmid pGBXN1, only the Nae I site downstream of the translation initiation codon ATG of the gB gene is converted into Cla I site.

The plasmid pGBXN1 (2 µg) obtained above is treated with Cla I (2 units) in a mixture (50 µl) of 6 mM Tris-HCl (pH 7.9), 50 mM NaCl, 6 mM $MgCl_2$ and 6 mM 2-mercaptoethanol at 37°C for one hour. The reaction mixture is subjected to treatment with phenol and precipitation with ethanol. The DNA thus obtained is reacted with $T_4$ polymerase and further with a Xho I linker (CCTCGAGG) in the same manner as described above. From the plasmids thus obtained, there is selected the desired plasmid pGBXN2 wherein the Cla I site is converted into a Xho I site.

(2) Preparation of shuttle vector pONY1:

Shuttle vector pAM 82 as prepared in the same manner as described in Japanese Patent First Publication No. 31799/1984 is treated as follows in order to convert the Puv II site into a BamHI site.

A fragment (2 µg) prepared by cleaving the plasmid pAM 82 with Xho I is reacted with $T_4$ DNA polymerase (0.1 unit) in a mixture (50 µl) of 67 mM Tris-HCl (pH 8.6), 6.7 mM $MgCl_2$, 10 mM 2-mercaptoethanol, 6.7 µM EDTA and 16.7 mM $(NH_4)_2SO_4$ which contains 200 µM dATP, dCTP, dGTP and dTTP at 37°C for 30 minutes. The reaction mixture is subjected to phenol extraction and ethanol precipitation. The resulting DNA is reacted with a BamHI Linker in a molar ratio of 1:10 by using $T_4$ ligase at 16°C for 8 hours.

E. coli x1776 is transformed with the reaction mixture obtained above in the same manner as described hereinbefore. The ampicillin-resistant cells thus obtained are incubated, and from the cells thus obtained, there is isolated a plasmid pONY1 in the same manner as described hereinbefore. In said plasmid pONY1, the Pvu II site of pAM 82 is converted into BamHI site.

(3) Preparation of HSVgB gene expression plasmid:

The plasmid pGBXN2 (10 µg) containing HSVgB gene as prepared above is reacted with BamHI (10 units) and Xho I (10 units) in a mixture (100 µl) of 6 mM Tris-HCl (pH 7.5), 6 mM $MgCl_2$, 6 mM 2-mercaptoethanol and 150 mM NaCl at 37°C for 2 hours. From the reaction mixture there is isolated a DNA fragment (2.9 kb) containing HSVgB gene without the sequence-encoding for the signal peptide by 1% agarose electrophoresis.

Separately, the shuttle vector pONY1 (10 µg) obtained in the above (2) is treated with restriction enzymes BamHI and Xho I in the same manner as described hereinbefore. From the reaction mixture there is isolated a DNA fragment (10 kb) containing the acid phosphatase promoter by 1% agarose electrophoresis.

The 2.9 kb fragment (100 ng) and the 10 kb fragment (10 ng) obtained above are reacted with $T_4$ ligase (0.1 unit) in a mixture (10 µl) of 66 mM Tris-HCl (pH 7.6), 6.6 mM $MgCl_2$, 10 mM dithiothreitol and 66 µM ATP at 16°C for 8 hours.

E. coli x1776 is transformed with the reaction mixture obtained above in the same manner as described hereinbefore. The ampicillin-resistant cells thus obtained are incubated, and from the cells thus obtained, there is isolated a recombinant plasmid pONYGB2 wherein the HSVgB gene without the sequence encoding the N-terminal 83 amino acids including the signal peptide-encoding region is inserted the downstream of the acid phosphatase promoter in the same manner as described hereinbefore.

(4) Preparation of transformed yeast:

The starting yeast is Saccharomyces cerevisiae AH 22 [a, leu 2, his 4, can 1 (Cir+)], which has been deposited at Fermentation Research Institute, Agency of Industrial Science and Technology, Japan under Budapest Treaty as "FERM BP—312". The starting yeast is inoculated in the YPD medium (100 ml) consisting of 2% polypeptone, 1% yeast extract and 2% glucose. The mixture is incubated at 30°C overnight, and thereafter, the cells are collected by centrifugation. The cells thus collected are washed with sterilized water (20 ml), suspended in a solution (5 ml) of 1.2 M sorbitol and Zymolyase-60,000 (manufactured by Seikagaku Kogyo K.K., Japan, 100 µg/ml), and the suspension is allowed to stand at 30°C for 30 minutes to give spheroplasts. The spheroplasts thus prepared are washed with 1.2 M sorbitol solution three times, and then suspended in a solution (0.6 ml) of 2 M sorbitol, 10 mM $CaCl_2$ and 10 mM Tris-HCl (pH 7.5). The suspension thus prepared is divided into a small test tube in a volume of 60 µl. To the suspension is added the solution of the recombinant plasmid pONYGB2 (10 µg) as prepared in the above (3). After mixing well, 0.1 M $CaCl_2$ (3 µl) is added thereto in a final concentration of 10 mM $CaCl_2$, and the mixture is allowed to stand at room temperature for 5 to 10 minutes. To the resulting mixture is added each 1 ml of a solution of 20% polyethylene glycol 4,000, 10 mM $CaCl_2$ and 10 mM Tris-HCl (pH 7.5), and the mixture is allowed to stand at room temperature for about 20 minutes. The resulting mixture (each 0.2 ml) is added to a medium (10 ml) consisting of 22% sorbitol, 2% glucose, 0.7% yeast nitrogen base amino acid, 2% YPD, 20 µg/ml histidine and 3% agar, which is kept at a constant temperature of 45°C. After gently mixing, the mixture is added in a layer onto a plate of minimal medium containing 1.2 M sorbitol which is previously prepared and consists of 0.7% yeast nitrogen base amino acid, 2% glucose, 20 µg/ml histidine and 2% agar and is set thereon. The plate is incubated at 30°C to give colonies of a leucine-non-requiring yeast. The colonies are incubated in a BurkHorlder minimal medium supplemented with histidine (20 µg/ml) [cf. Tohe, A. et al: J. Bacterol., 113, 727—738, 1973] to give the desired transformed yeast: Saccharomyces cerevisiae YGBS.

(5) Production of HSVgB by the transformed yeast:

The colony of the transformed yeast obtained in the above (4) is applied onto an agar plate of BurkHolder minimal medium supplemented with histidine (20 µg/ml) and incubated at 30°C to form a colony (in order to confirm the transformant requiring no leucine). The resulting cells are separated from the colony, inoculated in BurkHolder minimal medium (10 ml) supplemented with histidine (20 µg/ml) and incubated at 30°C. After about 24 hours, the cells in logarithmic growth phase are collected by centrifugation and are suspended in a minimal medium (10 ml) containing no phosphoric acid (which is prepared by replacing $KG_2PO_4$ in BurkHolder minimal medium with KCl, followed by supplementing 20 µ/ ml histidine) in a cell concentration of about $4 \times 10^6$ cells/ml. After incubating at 30°C for about 24 hours, the culture broth is centrifuged at 1700 x g for 10 minutes to collect the cells. The cells thus separated are suspended in a solution (3 ml) of 1.2 M sorbitol, 50 mM phosphate buffer (pH 7.2), 14 mM 2-mercaptoethanol and 100 µg/ml Zymolyase-60,000 (manufactured by Seikagaku Kogyo K.K., Japan)., and the mixture is gently shaken at 30°C for 30 minutes to give spheroplast. The spheroplast is collected by centrifugation and is well suspended in 50 mM phosphate buffer (pH 7.2, 1 ml) containing 1% Tritone X—100, and the mixture is vigorously stirred with glass beads to fracture the cells. The resulting fractured mixture is centrifuged at 2700 x g for 10 minutes, and the resulting supernatant is taken as the yeast-lysed solution. As to the supernatant, the gB antigen activity was measured by an enzyme immunoassay. The results are shown in Table 1. In Table 1, there are also shown the value as to the yeast having shuttle vector pONY1 as a negative control and the value of gB purified from HSV as a positive control. As is clear from the table, the yeast transformed with the recombinant plasmid of the present invention produces effectively the desired HSVgB.

Besides, the fractured solution obtained above (each 1 ml) was subcutaneously administered to guinea pigs (5 animals) four times in each other week. As a result, there was observed neutralizing antibody in all guinea pigs, by which it was confirmed that the fractured solution had gB immunogenicity.

In case of other recombinant plasmids pONYGB3 and pONYGB5, almost similar results were obtained.

TABLE 1

| Run No. | 1 | 2 | 3 | 4 | 5 | 6 | 7 | 8 |
|---|---|---|---|---|---|---|---|---|
| gB activity $(OD_{492})$ | 0.75 | 0.62 | 0.70 | 0.87 | 0.67 | 0.66 | 0.01 | 0.45 |

[Note]: Run Nos. 1—6: Yeast clone (TGBS) which were transformed by plasmid pONYGB2
Run No 7: Yeast having plasmid pONYG1
Run No. 8: HSV-origin purified gB

**Claims**

1. A recombinant plasmid which comprises a plasmid vector containing a yeast DNA sequence, an *Escherichia coli* DNA sequence and a promoter region and downstream and under the control of the promoter region a Herpes simplex virus gB gene having lost a part of the N-terminal sequences including the signal peptide-encoding region.

2. The recombinant plasmid according to claim 1, characterized in that the Herpes simplex virus gB gene is a gB gene without the sequence encoding the N-terminal 30 amino acids.

3. The recombinant plasmid according to claim 1, characterized in that the Herpes simplex virus gB gene is a gB gene without the sequence encoding the N-terminal 83 amino acids.

4. The use of a recombinant plasmid according to any one of claims 1 to 3 for preparing HSVgB in yeast.

5. Yeast transformed with a recombinant plasmid according to any one of claims 1 to 3.

6. Method for the production of HSVgB wherein a transformed yeast according to claim 5 is cultivated in a medium under suitable conditions, the HSVgB is accumulated in the culture and is then recovered therefrom.

**Patentansprüche**

1. Rekombinantes Plasmid enthaltend einen Plasmid-Vektor der eine Hefe-DNA-Sequenz aufweist, eine *Escherichia coli* DNA-Sequenz und einen Promotor-Bereich, und stromabwärts und unter der Kontrolle des Promotor-Bereichs ein Herpes simplex-Virus-gB-Gen, dem ein Teil der N-terminalen Sequenzen einschließlich des Signalpeptid-codierenden Bereichs fehlt.

2. Rekombinantes Plasmid nach Anspruch 1, dadurch gekennzeichnet, daß das Herpes simplex-Virus-gB-Gen ein gB-Genohne die Sequenz ist, die die N-terminalen 30 Aminosäuren codiert.

3. Rekombinantes Plasmid nach Anspruch 1, dadurch gekennzeichnet, daß das Herpes simplex-Virus-gB-Gen ein gB-Genohne die Sequenz ist, die die N-terminalen 83 Aminosäuren codiert.

4. Verwendung eines rekombinanten Plasmids nach einem der Ansprüche 1 bis 3 zur Herstellung von HSVgB in Hefe.

5. Hefe, die mit einem rekombinanten Plasmid nach einem der Ansprüche 1 bis 3 transformiert ist.

6. Verfahren zur Herstellung von HSVgB, bei dem eine transformierte Hefe nach Anspruch 5 in einem Medium unter geeigneten Bedingungen gezüchtet wird, HSVgB sich in der Kultur ansammelt und sodann daraus gewonnen wird.

**Revendications**

1. Un plasmide recombinant qui comprend un vecteur plasmide contenant une séquence d'ADN de levure, une séquence d'ADN *d'Escherichia coli* er une région de promoteur et en aval et sous le contrôle de la région de promoteur un gène gB du virus de l'herpès simplex ayant perdu une partie des séquences N-terminales incluant la région codant pour le peptide signal.

2. La plasmide recombinant selon la revendication 1, caractérisé en que ce le gène gB de virus de l'herpès simplex est un gène gB sans la séquence codant pour les 30 aminoacides N-terminaux.

3. La plasmide recombinant selon la revendication 1, caractérisé en ce que le gène gB de virus de l'herpès simplex est un gène gB sans la séquence codant pour les 83 aminoacides N-terminaux.

4. L'utilisation d'un plasmide recombinant selon l'une quelconque des revendications 1 à 3 pour la préparation du HSVgB dans la levure.

5. Levure transformée par un plasmide recombinant selon l'une quelconque des revendications 1 à 3.

6. Procédé pour la production du HSVgB selon lequel une levure transformée selon la revendication 5 est cultivée dans un milieu dans des conditions appropriées, le HSVgB s'accumule dans la culture et est ensuite récupéré à partir de celle-ci.

Fig. 1 (A)

1-CTCGAGTTGCGCCGCCCGGACTGCAGCCGCCCGACCTCCGAAGGTCGTTACCGTTACCCGCCCGGCGTATATCTCACGTACGACTCCGACTGTCCGCTGG

101-TGGCCATCGTCGAGAGCGCCCCCGACGGCTGTATCGGCCCCCGGTCGGTCGTGGTCTACGACGCCGACGTTTTCTCGATCCTCTACTCGGTCCTCCAGCA

201-CCTCGCCCCCAGGCTACCTGACGGGGGGGCACGACGGGCCCCCGTAGTCCCGCC

254-ATG CAC CAG GGC GCC CCC TCG TGG GGG CGC CGG TGG TTC GTC GTA TGG GCG CTC TTG GGG TTG ACG CTG GGG GTC
    Met-His-Gln-Gly-Ala-Pro-Ser-Trp-Gly-Arg-Arg-Trp-Phe-Val-Val-Trp-Ala-Leu-Leu-Gly-Leu-Thr-Leu-Gly-Val-25

329-CTG GTG GCG TCG GCG GCT CCG AGT TCC CCC GGC ACG CCT GGG GTC GCG CGC GAC CCA GGC GGC GAA CGG GGG CCC
    Leu-Val-Ala-Ser-Ala-Ala-Pro-Ser-Ser-Pro-Gly-Thr-Pro-Gly-Val-Ala-Arg-Asp-Pro-Gly-Gly-Glu-Arg-Gly-Pro-50

404-TGC CAC TCC GGC GCC GCC GCC CTT GGC GCC GCC CCA ACG GGG GAC CCG AAA CCG AAG AAG AAC AAA AAA CCG AAA
    Cys-His-Ser-Gly-Ala-Ala-Ala-Leu-Gly-Ala-Ala-Pro-Thr-Gly-Asp-Pro-Lys-Pro-Lys-Lys-Asn-Lys-Lys-Pro-Lys-75

                            Nae I
479-AAC CCA ACG CCA CCA CGC CCC GCC|GGC GAC AAC GCG ACC GTC GCC GCG GGC CAC GCC ACC CTG CGC GAG CAC CTG
    Asn-Pro-Thr-Pro-Pro-Arg-Pro-Ala-Gly-Asp-Asn-Ala-Thr-Val-Ala-Ala-Gly-His-Ala-Thr-Leu-Arg-Glu-His-Leu-100

554-CGG GAC ATC AAG GCG GAG AAC ACC GAT GCA AAC TTT TAC GTG TGC CCA CCC CCC ACG GGC GCC ACG GTG GTG CAG
    Arg-Asp-Ile-Lys-Ala-Glu-Asn-Thr-Asp-Ala-Asn-Phe-Tyr-Val-Cys-Pro-Pro-Pro-Thr-Gly-Ala-Thr-Val-Val-Gln-125

629-TTC GAG CAG CCG CGC CGC TGC CCG ACC CGG CCC GAG GGT CAG AAC TAC ACG GAG GGC ATC GCG GTG GTC TTC AAG
    Phe-Glu-Gln-Pro-Arg-Arg-Cys-Pro-Thr-Arg-Pro-Glu-Gly-Gln-Asn-Tyr-Thr-Glu-Gly-Ile-Ala-Val-Val-Phe-Lys-150

EP 0 216 195 B1

Fig. 1 (B)

704- GAG AAC ATC GCC CCG TAC AAG TTC AAG GCC ACC ATG TAC TAC AAA GAC GTC ACC GTT TCG CAG GTG TGG TTC GGC
Glu-Asn-Ile-Ala-Pro-Tyr-Lys-Phe-Lys-Ala-Thr-Met-Tyr-Tyr-Lys-Asp-Val-Thr-Val-Ser-Gln-Val-Trp-Phe-Gly-175

779- CAC CGC TAC TCC CAG TTT ATG GGG ATC TTT GAG GAC CGC GCC CCC GTC CCC TTC GAG GAG GTG ATC GAC AAG ATC
His-Arg-Tyr-Ser-Gln-Phe-Met-Gly-Ile-Phe-Glu-Asp-Arg-Ala-Pro-Val-Pro-Phe-Glu-Glu-Val-Ile-Asp-Lys-Ile-200

854- AAC GCC AAG GGG GTC TGT CGG TCC ACG GCC AAG TAC GTG CGC AAC AAC CTG GAG ACC ACC GCG TTT CAC CGG GAC
Asn-Ala-Lys-Gly-Val-Cys-Arg-Ser-Thr-Ala-Lys-Tyr-Val-Arg-Asn-Asn-Leu-Glu-Thr-Thr-Ala-Phe-His-Arg-Asp-225

929- GAC CAC GAG ACC GAC ATG GAG CTG AAA CCG GCC AAC GCC GCG ACC CGC ACG AGC CGG GGC TGG CAC ACC ACC GAC
Asp-His-Glu-Thr-Asp-Met-Glu-Leu-Lys-Pro-Ala-Asn-Ala-Ala-Thr-Arg-Thr-Ser-Arg-Gly-Trp-His-Thr-Thr-Asp-250

1004- CTC AAG TAC AAC CCC TCG CGG GTG GAG GCG TTC CAC CGG TAC GGG ACG ACG GTA AAC TGC ATC GTC GAG GAG GTG
Leu-Lys-Tyr-Asn-Pro-Ser-Arg-Val-Glu-Ala-Phe-His-Arg-Tyr-Gly-Thr-Thr-Val-Asn-Cys-Ile-Val-Glu-Glu-Val-275

1079- GAC GCG CGC TCG GTG TAC CCG TAC GAC GAG TTT GTG CTG GCG ACT GGC GAC TTT GTG TAC ATG TCC CCG TTT TAC
Asp-Ala-Arg-Ser-Val-Tyr-Pro-Tyr-Asp-Glu-Phe-Val-Leu-Ala-Thr-Gly-Asp-Phe-Val-Tyr-Met-Ser-Pro-Phe-Tyr-300

Sal I

1154- GGC TAC CGG GAG GGG TCG CAC ACC GAA CAC ACC ACG TAC GCC GCC GAC CGC TTC AAG CAG GTC GAC GGC TTC TAC
Gly-Tyr-Arg-Glu-Gly-Ser-His-Thr-Glu-His-Thr-Thr-Tyr-Ala-Ala-Asp-Arg-Phe-Lys-Gln-Val-Asp-Gly-Phe-Tyr-325

EP 0 216 195 B1

Fig. 1 (C)

GCG CGC GAC CTC ACC ACC AAG GCC CGG GCC ACG GCG CCG ACC ACC CGG AAC CTG CTC ACG ACC CCC AAG TTC ACC
Ala - Arg - Asp- Leu - Thr- Thr - Lys - Ala - Arg - Ala - Thr- Ala - Pro - Thr - Thr - Arg - Asn - Leu - Leu - Thr - Thr - Pro - Lys - Phe - Thr - 350

GTG GCC TGG GAC TGG GTG CCA AAG CGC CCG TCG GTC TGC ACC ATG ACC AAG TGG CAG GAA GTG GAC GAG ATG CTG
Val - Ala - Trp - Asp - Trp - Val - Pro - Lys - Arg - Pro - Ser - Val - Cys - Thr - Met - Thr - Lys - Trp - Gln - Glu - Val - Asp - Glu - Met - Leu - 375

CGC TCC GAG TAC GGC GGC TCC TTC CGA TTC TCC TCC GAC GCC ATA  TCC ACC ACC TTC ACC ACC AAC CTG ACC GAG
Arg - Ser - Glu - Tyr - Gly - Gly - Ser - Phe - Arg - Phe - Ser - Ser - Asp - Ala - I le - Ser - Thr - Thr - Phe - Thr - Thr - Asn - Leu - Thr - Glu - 400

EP 0 216 195 B1

Fig. 2

Fig. 3

Fig. 4

Fig. 5

Fig. 6